# EUROPÄISCHE PATENTSCHRIFT

(11) **EP 0 692 221 B1**
(45) Veröffentlichungstag und Bekanntmachung des Hinweises auf die Patenterteilung: **20.01.1999**
(21) Anmeldenummer: 95108948.1
(22) Anmeldetag: 10.06.1995
(51) Int. Cl.: A61B 5/042, A61N 1/05

(54) **Katheter mit Elektrode**
Catheter having electrodes
Cathéter avec électrodes

(30) Priorität: 16.07.1994 DE 4425195
(43) Veröffentlichungstag der Anmeldung: 17.01.1996
(73) Patentinhaber: Osypka, Peter, Dr. Ing., 79639 Grenzach-Wyhlen (DE)
(72) Erfinder: Osypka, Peter, Dr. Ing., 79639 Grenzach-Wyhlen (DE)
(74) Vertreter: Schmitt, Hans, Dipl.-Ing.

(56) Entgegenhaltungen:
- EP-A- 0 609 182
- WO-A-95/15115
- DE-A- 4 025 369
- GB-A- 2 032 278
- US-A- 5 255 679

## Beschreibung

Die Erfindung betrifft einen Katheter mit einem Führungsschlauch und mit einer oder mehreren elektrischen Zuleitungen zu einer Elektrode oder zu einer mehrere gegeneinander isolierte Elektroden oder Pole aufweisenden Mehrfachelektrode, die zum intrakardialen, insbesondere punktuellen Messen oder Aufnehmen von Herzsignalen oder elektrischen Potentialen im Herzen und/oder zum Stimulieren des Herzens dient.

Für die Auslösung von Tachykardien im Herzen werden veränderte Leitungsbahnen bzw. Zellgruppen verantwortlich gemacht. Zur Verhinderung solcher nicht selten lebensbedrohenden Tachykardien wird unter anderem die Zerstörung entsprechender Zellgruppen vorgenommen. Eine Methode dafür ist die Zerstörung durch Wärme mit Hilfe elektrischer Hochfrequenz-Energie. Diese Therapie ist unter dem Begriff Hochfrequenz-Ablation bekannt. Die Anwendung der Hochfrequenz-Energie ist seit vielen Jahren in der Chirurgie außerhalb des Herzens, zum Beispiel für die Koagulation, ebenfalls bekannt.

Zunächst ist es erforderlich, den Ort der Ursache für die Tachykardien im Herzen ausfindig zu machen. Dazu benötigt man einen mehrpoligen Elektroden-Katheter der eingangs erwähnten Art, um durch das sogenannte "Mapping" das intrakardiale Signal zu finden, welches eine Auskunft über den abartigen Leitungsmechanismus im Herzen gibt. Hierzu wird der Katheter der eingangs erwähnten Art über ein Blußgefäß (Vene oder Arterie) bis in die entsprechende Herzkammer eingeführt. Die Lokalisation der Tachykardie auslösenden Leitungsbahnen bzw. Zellgruppen erfordert dabei einen großen Zeitaufwand. Man bedient sich dazu mehrpoliger steuerbarer Elektroden, mit deren Hilfe das Herz innen "abgetastet" wird. Da eine solche Abtastung im Herzen häufig an Stellen durchgeführt werden muß, die besonders glatt sind, zum Beispiel im Bereich der Herzklappen oder an den Herzklappen, das heißt nur geringe Aussicht besteht, die Elektrodenspitzen des Katheters an diesem Ort zu fixieren, erfordert es besonders viel Geschick und Geduld, derartige Messungen durchzuführen.

Der Erfindung liegt deshalb die Aufgabe zugrunde, einen Katheter der eingangs erwähnten Art so auszubilden, daß die Elektroden oder Pole stabil auch an besonders glatten Bereichen der Innenseite des Herzens positioniert und fixiert werden können, so daß jede Kammer des Herzens auf einfache Weise vollständig abgetastet bzw. von allen Teilen des Herzen elektrisch intrakardiale Signale aufgenommen werden können. Gleichzeitig soll es möglich sein, die Elektrodenanordnung so auszugestalten, daß das Herz auch stimuliert und/oder ablatiert werden kann, wobei wiederum eine gute Positionierung und Fixierung erforderlich ist.

Zur Lösung dieser Aufgabe ist der eingangs erwähnte Katheter dadurch gekennzeichnet, daß an dem in das Herz einführbaren distalen Ende des Katheters wenigstens eine Schlinge aus biegsamem oder flexiblem Werkstoff angeordnet und durch Ausschieben aus dem Führungsschlauch des Katheters in ihrem Umfang vergrößerbar ist und daß diese Schlinge als Führungsdraht oder -faden und/oder als Halter für wenigstens einen ebenfalls aus dem Führungsschlauch ausschiebbaren weiteren Führungsdraht für die Elektrode oder Mehrfachelektrode dient.

Es kann also aus dem distalen Ende oder Kopf des Elektrodenkatheters eine Schlinge, bevorzugt eine Drahtschlinge ausgefahren werden, die so groß gemacht werden kann, daß sie an jeder beliebigen Stelle im Inneren des Herzens an der Wand positioniert werden kann, weil sie sich aufgrund ihrer Umfangszunahme gegen die Herzinnenwand anlegt und daran abstützt. So kann beispielweise die Schlinge im rechten Vorhof an dem Ring der rechten Ventrikelklappe fest in Position gebracht werden. Je nach anatomischer Lage kann sich die Schlinge den Gegebenheiten vollständig anpassen, da sie biegsam und flexibel ist.

Diese Schlinge kann als Führungsdraht oder -faden für die Elektrode oder Mehrfachelektrode dienen, die somit entlang der Wand des Herzens, welche die Schlinge berührt, ausgefahren werden kann. Die Elektroden oder Pole können dann elektrische Signale aufnehmen, so daß die Herzinnenwand entlang der Schlinge bequem abgetastet werden kann.

Um beispielsweise den gesamten Herzklappenring elektrisch abzutasten, kann die Elektrode oder Mehrfachelektrode über die gesamte Schlinge, also praktisch über 360° geschoben werden. Handelt es sich dabei beispielsweise um eine achtpolige oder zehnpolige isolierte Metallwendel und diese distal an bestimmten Stellen blank, also abisoliert, kann mann aus der Position der Wendel und dem bekannten Abstand der Pole voneinander nach und nach durch Zurückziehen der Wendel den gesamten Herzklappenring umfahren und dort jeweils die elektrischen Signale aufnehmen. Das flexible biegsame Material der Schlinge beeinflußt dabei in vorteilhafter Weise nicht die Bewegung und Pumpwirkung des Herzens, ist aber steif genug, um der Schlinge die erforderliche Stabilität zum Fixieren und Positionieren im Herzen zu geben und als Führungsdraht dienen zu können. Dabei sind als Material nicht nur Drähte, sondern auch Kunststoffmonophile geeignet. Die Schlinge und/oder der oder die daran gehaltenen Führungsdrähte können also aus flexibel biegsamem, an die Herzinnenseite anpaßbarem, nicht bleibend verformbarem Draht, zum Beispiel aus Nitinol, oder aus Kunststoff bestehen.

Eine Alternative oder zusätzliche Möglichkeit besteht gemäß dem zweiten Teil der Lösung der Aufgabe darin, daß die Schlinge als Halter für wenigstens einen weiteren, aus dem Führungsschlauch ausschiebbaren Führungsdraht für eine Mehrfachelektrode dient. Ein solcher weiterer Führungsdraht kann also mit der Schlinge ein Raumgebilde herstellen, welches sich noch besser an der Herzinnenwand anlegen und abstützen läßt, so daß die Mehrfachelektrode auch über den weiteren Führungsdraht vorgeschoben und zum Abtasten entsprechender Wandteile benutzt werden kann. Somit entfällt eine aufwendige Sucharbeit mit steuerbaren Elektroden.

Besonders zweckmäßig ist es, wenn die Schlinge und/oder der weitere Führungsdraht stufenlos verschiebbar und dadurch in ihrer wirksamen Länge stetig vergrößerbar sind. Auf diese Weise kann das als Führungselement für Mehrfachelektroden dienende Raumgebilde aus Schlinge und Führungsdraht an beliebige anatomische Verhältnisse in unterschiedlichen Herzen angepaßt werden und erlaubt eine gute Abtastung der Innenseite verschiedener Herzbereiche. Vor allem kann dabei auch die Lage der Schlinge und/oder des Führungsdrahtes innerhalb des Herzens für mehrere unterschiedliche Abtastungen auf einfache Weise verändert werden.

Die Elektrode oder Mehrfachelektrode kann zweckmäßigerweise eine Wendel aus einem oder mehreren Drähten sein, die in ihrem Inneren einen Kanal für den Führungsdraht freilassen, gegeneinander isoliert sind und am Endbereich abisolierbare oder abisolierte Stellen haben, wobei der Querschnitt des inneren Kanales der Elektrode oder Mehrfachelektrode gleich oder größer als der Außenquerschnitt des die Schlinge bildenden Drahtes oder Fadens oder des weiteren Führungsdrahtes oder -fadens ist, und dabei kann die Wendel und insbesondere ihr Ende auf der Schlinge und/oder dem weiteren Führungsdraht verschiebbar sein.

Es wurde bereits erwähnt, daß durch diese Merkmale und Maßnahmen die als Pole abisolierten Stellen praktisch stufenlos an verschiedene Herzbereiche entlang der Schlinge oder des Führungsdrahtes gebracht werden können, um jeweils Herzsignale punktuell messen oder aufnehmen zu können oder auch elektrische Potentiale im Herzen festzustellen. Die gegeneinander isolierten Drähte der Wendel können dabei jeweils die einzelnen Pole an ihren distalen Enden haben.

Eine konstruktiv besonders günstige Lösung ergibt sich, wenn die Schlinge mit einem Ende an der Innenseite des Führungsschlauches des Katheters befestigt ist und ihr anderes Ende durch den Katheter und dessen Führungsschlauch zum proximalen Katheterende verläuft und wenn die Elektrode oder Mehrfachelektrode von dem proximalen Katheterende aus bis zu der Befestigungsstelle der Schlinge verschiebbar ist. Zum Vergrößern des Umfanges der Schlinge kann also der unbefestigte Teil der Schlinge relativ zu dem Führungsschlauch verschoben werden und außerdem kann auf diese Weise die jeweilige Elektrode vom proximalen Katheterende aus auf die Schlinge geschoben und relativ zu ihr verschoben werden.

Um die Schlinge im Herzvorhof beispielsweise um die von der Herzklappe verschließbare Öffnung des Vorhofes herum bequem anordnen zu können, ist es zweckmäßig, wenn die Schlinge insbesondere an ihrer distalen Befestigungsstelle eine Vorspannung oder Verbiegung hat, durch welche die von der Schlinge aufgespannte Ebene in einem Winkel zum Katheterverlauf, insbesondere in einem stumpfen Winkel, angeordnet ist. Wird die Schlinge mit Hilfe des Führungsschlauches in einen Vorhof des Herzens eingeführt und dann ausgefahren, ergibt sich praktisch von selbst eine Abstützung entlang der Herzklappe, statt daß die Schlinge durch aufwendige Manipulationen noch in eine Lage quer zu der Zuführrichtung und zu dem Führungsschlauch gebracht werden müßte. Dennoch ist das Einführen der Schlinge durch ein Blutgefäß nicht erschwert, da die Schlinge zunächst ganz oder weitgehend in den Führungsschlauch eingezogen sein kann, wobei sich ihre Abbiegung kaum bemerkbar macht. Wird dann die Schlinge an dem gewünschten Ort beispielsweise im Bereich der Herzklappe ausgefahren, orientiert sie sich automatisch oder selbsttätig quer zu dem Führungsschlauch und kann entsprechend einfach positioniert werden.

Damit die Schlinge einerseits mit ihrem Ende fixiert und mit dem gegenüberliegenden Ende verschiebbar sein kann, ist es zweckmäßig, wenn am distalen Ende des Führungsschlauches ein Austrittskanal für das verschiebbare Ende der Schlinge exzentrisch und etwa auf einem Durchmesser gegenüberliegend eine Eintrittsöffnung mit der Befestigungsstelle für das feste Ende der Schlinge angeordnet sind. Somit kann die Befestigungsstelle in den Führungsschlauch verlegt sein, also beim Vorschieben des Führungsschlauches durch ein Blutgefäß und beim Zurückziehen nicht stören.

Es wurde schon erwähnt, daß die Schlinge auch als Halterung für einen weiteren Führungsdraht dienen kann. Besonders zweckmäßig ist es dabei, wenn der weitere Führungsdraht im Winkel zu der von der Schlinge aufgespannten Ebene proximal zu dem Austritt der Schlinge aus dem Führungsschlauch austritt und ausschiebbar ist und mit einer Führung, insbesondere einer Öse, an dem die Schlinge bildenden Führungsdraht oder -faden angreift und/oder darauf verschiebbar ist. Vor allem eine Verschiebung dieses weiteren Führungsdrahtes eröffnet entsprechend weitere Möglichkeiten, diesen Führungsdraht im Inneren des Herzens variabel zu positionieren, also mit festliegender Schlinge unterschiedliche Bereiche der Herzinnenwand "oberhalb" dieser Schlinge abzutasten und zu untersuchen. Über diesen weiteren Führungsdraht kann in schon erwähnter Weise eine Mehrfachelektrode mit ihren verschiedenen Polen geschoben werden, um entlang diesem weiteren Führungsdraht entsprechende Messungen oder auch Stimulationen oder dergleichen durchzuführen.

Es sei an dieser Stelle erwähnt, daß statt der zum Messen dienenden Elektrode oder Mehrfachelektrode natürlich sowohl auf der Schlinge als auch auf dem weiteren Führungsdraht auch eine Elektrode zum Stimulieren verschoben werden kann, wobei eventuell dieselbe, zum Messen dienende Elektrode auch zum Stimulieren herangezogen werden kann.

Zum Verschieben der Führung oder Öse des weiteren Führungsdrahtes entlang der Schlinge kann eine diese Führung beaufschlagende, über die Schlinge schiebbare, biegbare Hülse oder Wendel vorgesehen sein. Die Schlinge dient somit auch beim Steuern des Führungsdrahtes ihrerseits indirekt als Führung für diese biegbare Hülse oder Wendel, mit welcher der Führungsdraht gegenüber seinem Austritt aus dem Führungsschlauch praktisch verschwenkt werden kann, indem sein diesem Austritt aus dem Führungsschlauch entgegengesetztes Ende entlang der Schlinge verschoben wird, wobei die Schlinge wiederum auf unterschiedliche Umfänge gebracht werden kann. Die über die Schlinge schiebbare Hülse ist dabei zweckmäßigerweise ein biegbarer Schlauch.

Eine weitere Ausgestaltungsmöglichkeit kann darin bestehen, daß die zum Verstellen des weiteren Führungsdrahtes dienende Hülse oder Wendel ihrerseits mehrere Pole aufweist oder als Mehrfachwendel mit mehreren gegeneinander isolierten Elektroden oder Polen ausgebildet ist. Es kann dann sowohl im Bereich der Schlinge als auch im Bereich des weiteren Führungsdrahtes gemessen oder stimuliert werden.

Der weitere Führungsdraht hat dabei einen Querschnitt, der gleich oder kleiner als der Innenquerschnitt eines inneren Kanales in einer weiteren elektrischen Zuleitung mit insbesondere mehreren Elektroden oder Polen, vorzugsweise einer Mehrfachwendel, ist. Somit kann eine solche Mehrfachwendel gut über diesen weiteren Führungsdraht vorgeschoben und auch wieder zurückgeschoben werden.

Damit die Anordnung auf einfache Weise auch zum Ablatieren, das heißt zum Zerstören kranker Zellen an der Innenseite des Herzens herangezogen werden kann, sowie diese durch den vorhergehenden Meßvorgang ermittelt sind, kann wenigstens eine der Elektroden oder Pole eine Zuleitung für Hochfrequenz-Energie haben oder es kann ein zusätzlicher Hochfrequenz-Pol zum Koagulieren von Gewebe vorgesehen sein. Die Erfindung ermöglicht also eine Ausgestaltung dahingehend, daß über die gleiche Elektrodenanordnung, mit welcher elektrische intrakardiale Signale aufgenommen werden, das Herz auch an entsprechenden Stellen ablatiert werden kann.

In diesem Zusammenhang ist es vorteilhaft, wenn wenigstens ein Temperaturfühler am oder im Hochfrequenz-Pol angeordnet ist und insbesondere zur Regelung der Hochfrequenz mit einem diesen Hochfrequenz-Pol beaufschlagenden Hochfrequenz-Generator verbunden ist. Somit kann verhindert werden, daß beim Ablatieren zu hohe Temperaturen auftreten. Die entsprechende Energie kann gemäß der jeweils durch den Temperaturfühler festgestellten Erwärmung auf einen zum Ablatieren ausreichenden Wert geregelt werden, der aber die Umgebung des Ablationsvorganges nicht schädigt.

Eine weitere Ausgestaltung der Erfindung kann darin bestehen, daß zwei Schlingen vorgesehen sind und daß die Ebenen, in denen diese Schlingen angeordnet sind, in einem Winkel zueinander stehen. Dabei können die Schlingen in Orientierungsrichtung des Katheters gesehen etwas übereinander, aber im Winkel zueinander oder auch nach entgegengesetzten Seiten von der Mitte des Führungsschlauches des Katheters abstehend vorgesehen sein. Vor allem in Orientierungsrichtung des Katheters etwas übereinanderliegende Schlinge ergeben eine noch bessere Abstützmöglichkeit und darüber hinaus eine Vergrößerung der Möglichkeiten, Meß-Elektroden oder -Pole zuzuführen oder auch zu stimulieren und/oder zu ablatieren.

Besonders günstig ist es, wenn die beiden Schlingen an derselben Stelle des Katheters und nach derselben Seite hin unter verschiedenen Winkeln austreten. Sie können dann einzeln oder gemeinsam eine gute Fixierung an der Innenwand einer Herzkammer oder eines Herzvorhofes bewirken.

Dabei kann die unter einem kleineren Winkel gegenüber dem Katheter geneigte, dem Katheterende nähere Schlinge als Halter für den weiteren Führungsdraht oder ein auf der Schlinge verschiebbares Halteelement für den weiteren Führungsdraht dienen. Es ergibt sich also die Möglichkeit, mit der einen Schlinge einen oder mehrere Führungsdrähte zu halten gegebenenfalls verschiebbar zu halten, und mit der anderen Schlinge neben einer Fixierung auch eine Führung für eine Mehrfachwendel durchzuführen. Darüber hinaus bewirken zwei Schlingen aufgrund der Elastizität ihres Werkstoffes eine entsprechend gute Fixierung des gesamten Katheters im Inneren des Herzens, welches entsprechende Pumpbewegungen durchführt.

Eine weitere Ausgestaltung der Erfindung kann darin bestehen, daß wenigstens zwei weitere Führungsdrähte vorgesehen sind, deren dem Katheter abgewandte Enden an derselben Schlinge oder verschiedenen Schlingen gehalten, insbesondere verschiebbar gehalten sind und deren Angriffsstellen an der/den Schlingen einen Abstand zueinander haben. Ist nur eine Schlinge vorhanden, sind beide weiteren Führungsdrähte auf jeden Fall an derselben Schlinge befestigt. Sind zwei Schlingen vorgesehen, können die weiteren Führungsdrähte dennoch an derselben Schlinge, aber auch an den verschiedenen Schlingen angreifen. In jedem Fall ergibt sich dadurch ein Raumgebilde mit mehreren flexiblen Drähten, die korbartig an der Herzinnenseite abgestützt werden können. Darüber hinaus erhöht sich die Möglichkeit, ohne neue Positionierung unterschiedliche Bereiche an der Herzinnenseite zu messen und intrakardiale Signale aufzunehmen.

Der weitere Führungsdraht oder wenigstens einer von mehreren weiteren Führungsdrähten kann als hohler Katheter ausgebildet sein und ein offenes Ende haben, so daß durch diesen hohlen Katheter eine Elektrodenzuleitung mit einer oder mehreren Elektroden oder Polen an der Halterung dieses hohlen Katheters vorbei verschiebbar ist. Dies eröffnet die Möglichkeit, eine Schlinge im Herzvorhof auf der Herzklappe zu positionieren, dann aber mit Hilfe des hohlen weiteren Führungsdrahtes eine Mehrfachelektrode in die Hauptkammer des Herzens zu verschieben und dort Messungen oder Therapien oder Stimulationen durchzuführen.

Dabei ist es besonders günstig, wenn die durch den hohlen Katheter ausschiebbare Elektrodenzuleitung ein umgebogenes freies Ende hat und etwa J-förmig gestaltet ist, so daß das freie, die Pole oder Elektroden aufweisende Ende dieser Elektrodenzuleitung an der sich auf den Herzklappen abstützenden Schlinge vorbei verschiebbar ist und mit dem Ende die Unterseite der Herzklappen erreichbar ist. Gerade die Unterseiten der Herzklappen sind bisher nur sehr schwierig mit Hilfe von Elektroden zu erreichen, so daß dort das Aufnehmen von Herzsignalen oder elektrischen Potentialen bisher besonders schwierig ist. Durch die vorerwähnte Ausgestaltung der Erfindung kann nun auf der Herzklappe eine Abstützung und Festlegung des Führungsschlauches des Katheters erfolgen und dann über den weiteren Führungsdraht eine Elektrodenzuleitung unter die Herzklappen geführt werden.

Vor allem bei Kombination einzelner oder mehrerer der vorbeschriebenen Merkmale und Maßnahmen ergibt sich ein Katheter mit einem Führungsschlauch und einer oder mehreren Elektroden oder Mehrfachelektroden, die stabil in praktisch jedem beliebigen Teil des Herzens positioniert und fixiert werden kann, so daß jede Kammer des Herzens und auch der Vorhof auf einfache Weise vollständig abgetastet und von allen Teilen des Herzens elektrische intrakardiale Signale aufgenommen werden können. Darüber hinaus ist diese Katheterausbildung dazu geeignet, das Herz auch zu stimulieren oder Ablationen vorzunehmen. Trotzdem kann dieser Katheter sehr einfach durch ein Blutgefäß zugeführt werden, weil die zur Abstützung dienende Schlinge oder die Schlingen zunächst eingezogen sehr wenig Platz beanspruchen, dann aber am Ort der Positionierung so ausgeschoben werden können, daß sie sich innenseitig an der Herzwand anlegen und eine gewisse Klemmkraft oder auch eine formschlüssige Verbindung mit entsprechenden anatomischen Bereichen des Herzens bilden können, so daß die Pumpwirkung und Pumpbewegung des Herzens die Positionierung nicht gefährdet. Somit kann der Zeitaufwand zum intrakardialen, insbesondere punktuellen Messen und Aufnehmen von Herzsignalen und auch zum Stimulieren und Ablatieren in vorteilhafter Weise vermindert werden. Da eine Schlinge aus Nitinol bereits ausreicht, um die gewünschten Materialeigenschaften für eine sichere Positionierung bereitzustellen, ist die gesamte Anordnung auch preiswert.

Nachstehend sind Ausführungsbeispiele der Erfindung anhand der Zeichnung näher beschrieben. Es zeigt in zum Teil schematisierter Darstellung:
- Fig.1: eine Ansicht eines ersten Ausführungsbeispieles eines erfindungsgemäßen Katheters mit einer am distalen Ende eines Führungsschlauches ausgefahrenen, etwas zur Seite gebogenen Schlinge, die auf den Herzklappen einer Herzkammer im Herzvorhof abgestützt und positioniert ist,
- Fig.2: eine Seitenansicht des distalen Endes des Katheters mit daraus vorstehender Schlinge mit Blick genau in Richtung der von der Schlinge aufgespannten Ebene, die in einem stumpfen Winkel zum Katheterverlauf angeordnet ist,
- Fig.3: eine gegenüber Fig.2 um 90° gedrehte Ansicht des distalen Endes des Katheters mit daraus hervorstehender Schlinge,
- Fig.4: in vergrößertem Maßstab und teilweise im Längsschnitt das distale Ende des Katheters und seines Führungsschlauches mit der dort mit einem Ende befestigten Schlinge, auf welcher eine mehrere gegeneinander isolierte Elektroden oder Pole aufweisende Mehrfachelektrode in Form einer Mehrfachwendel aufgeschoben ist, die relativ zu der Schlinge verschiebbar ist, um unterschiedliche Meßpunkte innerhalb des Herzens berühren zu können,
- Fig.5: das proximale Ende des Katheters mit dem proximalen Ende der Schlinge, welches einen Handgriff zum Verschieben aufweist und in einen weiteren Handgriff eintritt, mit welchem die Mehrfachwendel relativ zu dem Führungsdraht verschiebbar ist, der die Schlinge bildet, und mit dem distalen Ende des Führungsschlauches, an welchem ein Anschlag für die Handgriffe angeordnet ist, um deren Verschiebung zu begrenzen,
- Fig.6: eine der Fig.1 entsprechende Darstellung, bei welcher ein weiterer Führungsdraht aus dem Führungsschlauch ausschiebbar und an der Schlinge befestigt ist und sich an der Innenwand des Herzvorhofes abstützt,
- Fig.7: einen Katheter mit Schlinge und weiterem Führungsdraht, der durch den Vorhof hindurch in die Herzkammer eingeführt und dort mittels des weiteren Führungsdrahtes und der entsprechend weit ausgefahrenen Schlinge positioniert und festgelegt ist,
- Fig.8: eine der Fig.7 entsprechende Darstellung, wobei der Katheter eine zweite Schlinge aufweist, durch welche die Positionierung innerhalb der Herzkammer verbessert ist,
- Fig.9: ein Ausführungsbeispiel mit einer Schlinge, an welcher ein Halter für einen hohlen weiteren Führungsdraht gehalten und insbesondere verschiebbar ist, aus welchem eine J-förmige Elektrodenzuleitung herausschiebbar ist, die unter die Herzklappe greift,
- Fig.10: eine Seitenansicht und
- Fig.11: eine Draufsicht einer Kombination einer am distalen Ende des Katheters befindlichen Schlinge mit einem weiteren Führungsdraht, auf welchem eine Mehrfachelektrode verschiebbar ist und dessen an der Schlinge angreifendes Ende seinerseits relativ zu der Schlinge verschiebbar ist, sowie
- Fig.12: eine Seitenansicht und
- Fig.13: eine Draufsicht einer abgewandelten Ausführungsform, bei welcher an der Schlinge zwei weitere Führungsdrähte mit Abstand zueinander angreifen.

Bei den nachstehend beschriebenen verschiedenen Ausführungsbeispielen eines im ganzen mit 1 bezeichneten Katheters werden übereinstimmende Teile auch bei abgewandelter Gestaltung, aber übereinstimmender Funktion mit gleichen Bezugszahlen benannt.

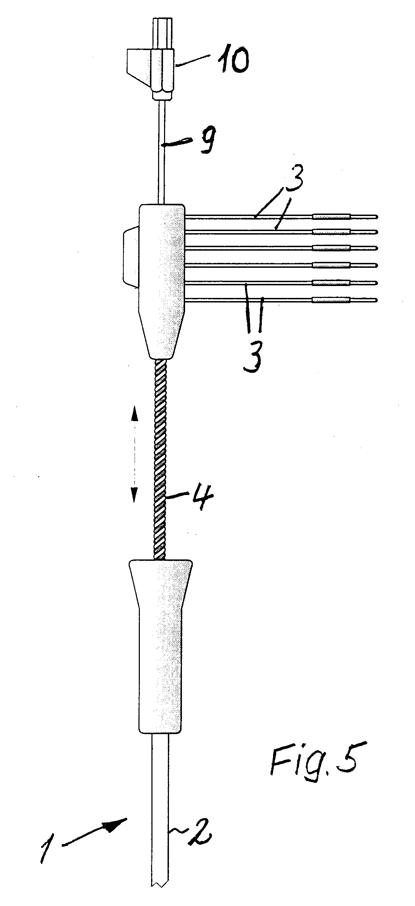

Der Katheter 1 hat bei allen Ausführungsbeispielen einen Führungsschlauch 2 und gemäß Fig.5 mehrere elektrische Zuleitungen 3 zu einer mehrere gegeneinander isolierte Elektroden oder Pole aufweisenden Mehrfachelektrode 4, deren proximales Ende in Fig.5 und deren distales Ende in Fig.4 erkennbar ist, wobei aber auch eine einfache Elektrode vorgesehen und in gleicher Weise angeordnet sein könnte. Die Pole der Elektrode 4 können dadurch gebildet sein, daß an dem distalen Endbereich die Isolierung der einzelnen Zuleitungen 3 punktuell so entfernt sind, daß blanke Stellen gebildet sind, die aber weiterhin gegeneinander isoliert sind, die aber in Berührkontakt mit dem Inneren des Herzens treten können. Dadurch kann der Katheter 1 zum intrakardialen, insbesondere punktuellen Messen oder Aufnehmen von Herzsignalen oder von elektrischen Potentialen im Herzen herangezogen werden. Darüber hinaus ist in noch zu beschreibender Weise auch eine Ablation im Inneren des Herzens oder eventuell eine Stimulation möglich.

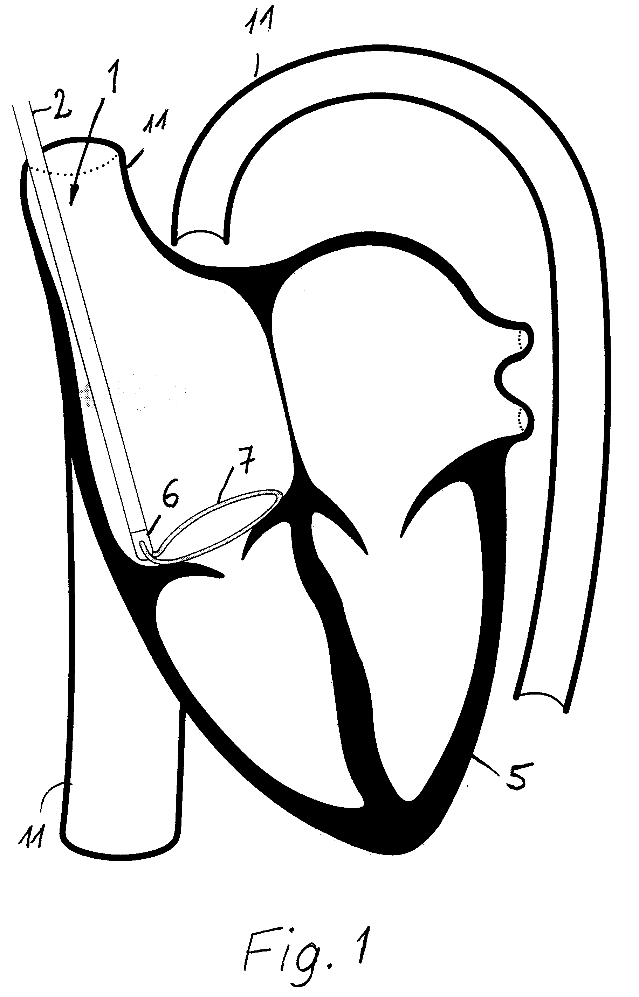

Bei allen Ausführungsbeispielen ist vorgesehen, daß an dem in das Herz 5 einführbaren distalen Ende 6 des Katheters 1 wenigstens eine Schlinge 7 aus biegsamem oder flexiblem Werkstoff angeordnet und durch Ausschieben aus dem Führungsschlauch 2 des Katheters 1 (vgl. auch Fig.4) in ihrem Umfang vergrößerbar ist. Gemäß Fig.4 dient diese Schlinge 7 im Ausführungsbeispiel gemäß Fig.1 bis 5, eventuell auch in den weiteren Ausführungsbeipielen als Führungsdraht oder -faden für die Mehrfachelektrode 4. Ebenso könnte sie auch als Führung für eine einfache Elektrode dienen.

Gemäß Fig.4 ist die Schlinge 7 mit einem Ende 8 an der Innenseite des Führungsschlauches 2 bzw. des Endes 6 des Katheters 1 befestigt. Ihr anderes Ende 9 verläuft gemäß Fig.5 durch den Katheter und dessen Führungsschlauch 2 zum proximalen Katheterende und tritt dort aus, so daß es mit Hilfe eines Handgriffes 10 relativ zu dem Führungsschlauch 2 verschoben werden kann, also die Schlinge 7 durch eine Verschiebung des Handgriffes 10 vergrößert oder verkleinert werden kann. Sie kann dabei so stark verkleinert werden, daß das Einführen des Katheters 1 durch Blutgefäße 11 praktisch nicht behindert werden kann, jedoch kann die Schlinge 7 auch so stark vergrößert werden, daß sie sich gemäß Fig.1 und 6 bis 9 gut im Herzen 5 an Innenwandbereichen anlegen und andrücken läßt, um den Katheter 1 zu fixieren und zu positionieren.

Die Mehrfachelektrode 4 verläuft ebenfalls vom proximalen Katheterende bis zu der Schlinge 7 und kann bis zu der Befestigungsstelle 12 der Schlinge 7 verschoben werden, also praktisch über 360°, so daß alle von der Schlinge 7 beaufschlagten Stellen der Herzinnenwand auch mit der Mehrfachelektrode 4 und daran vorgesehenen Polen erreichbar sind. Somit können punktuelle Messungen entlang der Schlinge 7 beispielsweise durch allmähliches Zurückziehen der Mehrfachelektrode 4 und der daran vorgesehenen Pole durchgeführt werden. Die Schlinge und auch ein in weiteren Ausführungsbeispielen vorgesehener, noch näher zu erläuternder weiterer Führungsdraht 13 sind dabei stufenlos verschiebbar, also in ihrer wirksamen Länge stetig vergrößerbar.

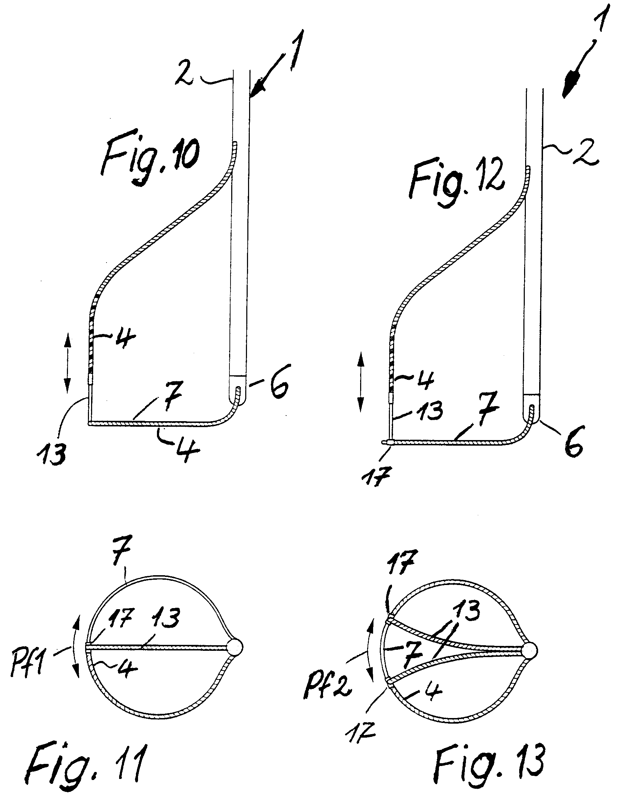

Anhand der Fig.4 und 5 erkennt man, daß die Mehrfachelektrode 4 eine Mehrfachwendel aus mehreren Drähten ist, die von den Zuleitungen 3 gebildet sind oder diese fortsetzen, wobei die Mehrfachelektrode 4 als Mehrfachwendel in ihrem Inneren einen Kanal für einen Führungsdraht freilassen, den die Schlinge 7 gemäß Fig.4 oder auch ein weiterer Führungsdraht 13 gemäß Fig.10 bis 13 bilden. Wie bereits erwähnt, sind die einzelnen Drähte gegeneinander isoliert und am Endbereich bereichsweise abisoliert, um entsprechende Elektroden oder Pole zum punktuellen Messen oder Aufnehmen von Herzsignalen oder elektrischen Potentialen im Herzen 5 zu ermöglichen. Anhand der Fig.4 wird deutlich, daß der Querschnitt des erwähnten inneren Kanales der Mehrfachelektrode gleich oder - im Ausführungsbeispiel - größer als der Außenquerschnitt des die Schlinge 7 bildenden Drahtes oder Fadens oder auch des weiteren Führungsdrahtes 13 ist, so daß die Mehrfachwendel 4 und insbesondere ihr Ende auf der Schlinge 7 - bzw. auf dem weiteren Führungsdraht 13 - verschiebbar ist.

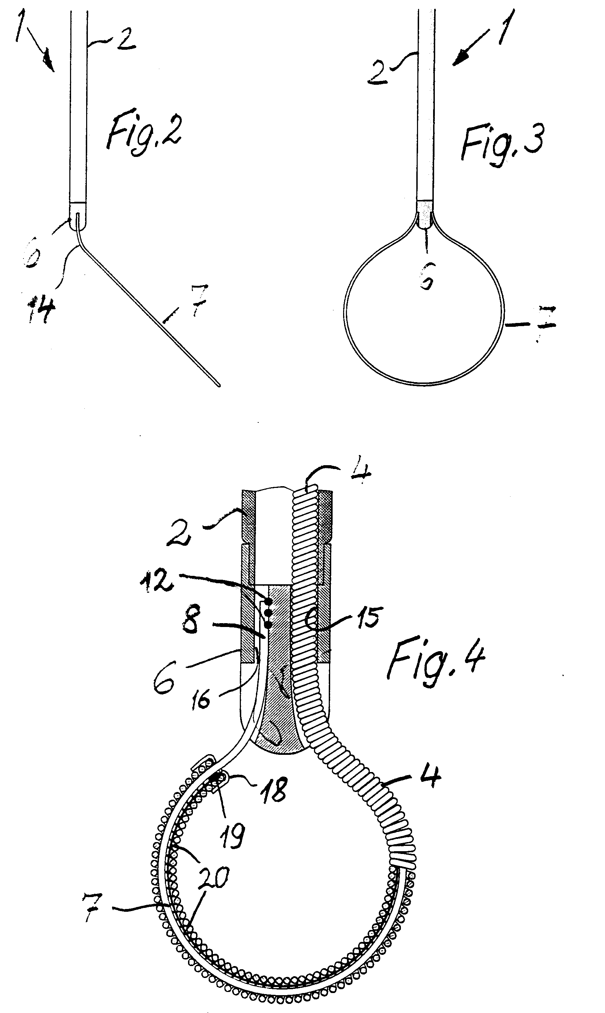

Damit die Schlinge 7 in der beispielsweise in Fig.1 dargestellten Form im Inneren des Herzens 5 bequem plaziert werden kann, ist gemäß Fig.2 vorgesehen, daß sie an dem distalen Ende 6 des Katheters und ihrer eigenen distalen Befestigungsstelle 12 eine Vorspannung und Verbiegung hat, durch welche die von der Schlinge 7 aufgespannte Ebene in der in Fig.2 ersichtlichen Weise in einem Winkel zum Katheterverlauf, in diesem Fall in einem stumpfen Winkel, angeordnet ist. In zurückgezogenem Zustand wirkt sich dies praktisch nicht aus, jedoch wird durch die Vorbiegung 14 beim Ausschieben der Schlinge 7 die in Fig.2 ersichtliche Position erreicht, die es erleichtert, beim Einschieben des Katheters 1 beispielsweise in einen Vorhof des Herzens 5 die Schlinge 7 auf den Herzklappen zu plazieren, ohne daß dies aufwendiger und komplizierter Bewegungen des Führungsschlauches 2 bedarf. Beim entsprechenden weiteren Ausschieben wird die Schlinge 7 dann von selbst eingeklemmt und positioniert und auch der Führungsschlauch 2 entsprechend seitlich im Herzen festgelegt.

Damit auf engem Raum einerseits die Befestigung der Schlinge 7 an der Stelle 12 innerhalb des distalen Endes 6 des Führungsschlauches 2 erfolgen kann und andererseits sowohl die Schlinge 7 als auch die demgegenüber verschiebbare Mehrfachelektrode 4 bequem ein- und ausgeschoben werden können, sind gemäß Fig.4 am distalen Ende 6 des Führungsschlauches 2 ein Austrittskanal 15 für das verschiebbare Ende der Schlinge 7 exzentrisch und etwa auf einem Durchmesser gegenüberliegend eine Eintrittsöffnung 16 mit der Befestigungsstelle 12 für das feste Ende 8 der Schlinge 7 angeordnet. Dies ergibt die in Fig.4 dargestellte einfache und platzsparende Befestigung des Endes 8 der Schlinge 7 bei gleichzeitiger Verschiebbarkeit des übrigen Schlingenteiles, so daß die Schlinge 7 sehr einfach durch Vorschieben und Zurückziehen ihres distalen Endes 9 in ihrem Durchmesser oder Umfang vergrößert und verkleinert werden kann, so daß auch die darüber geschobene Mehrfachelektrode 4 entsprechend geführt wird.

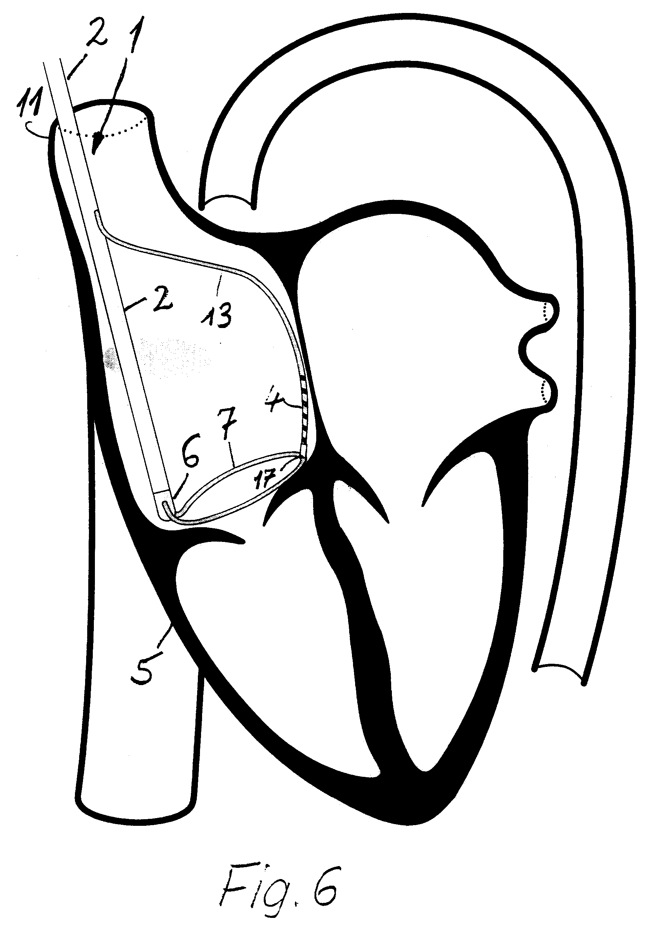

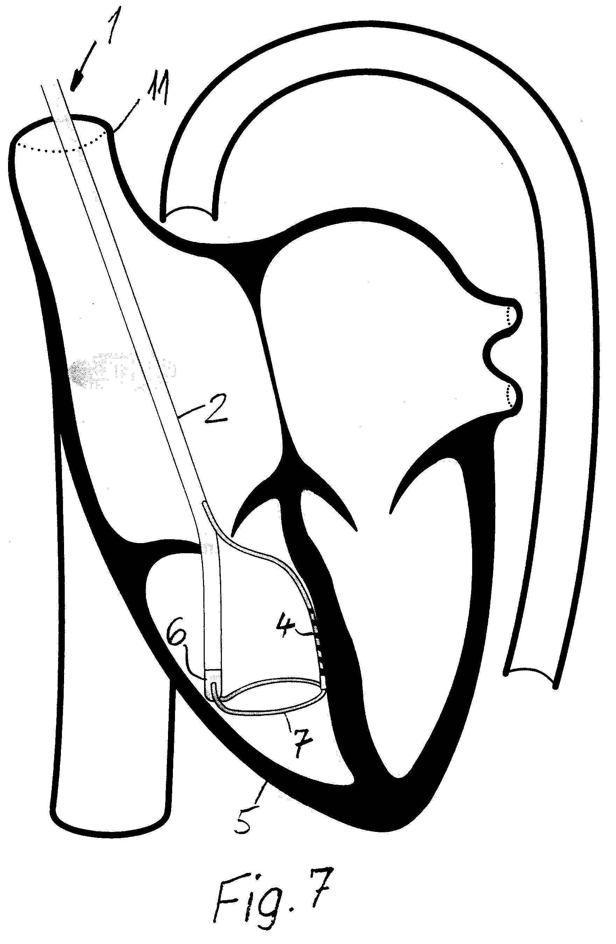

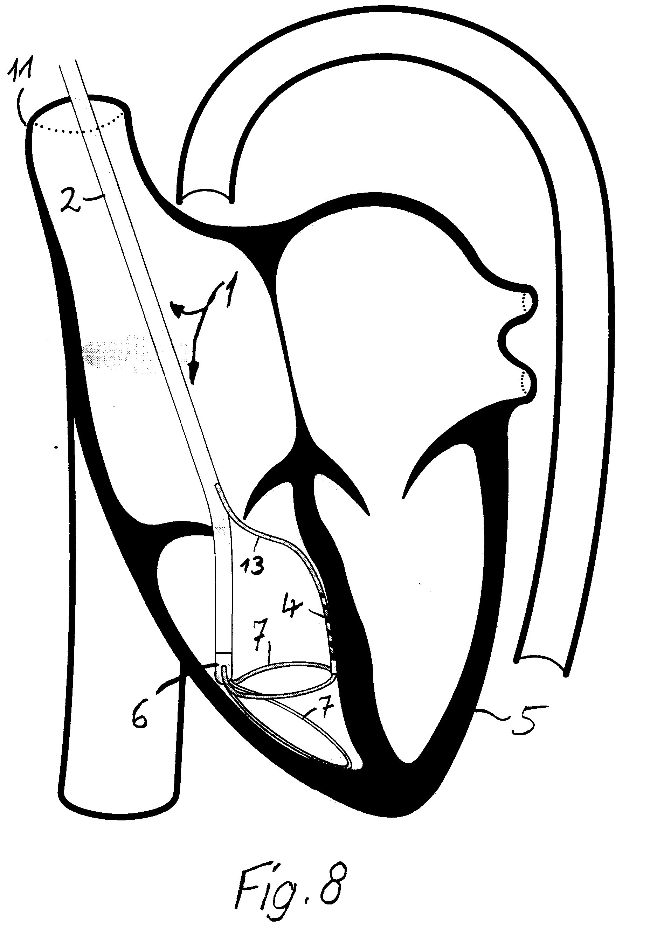

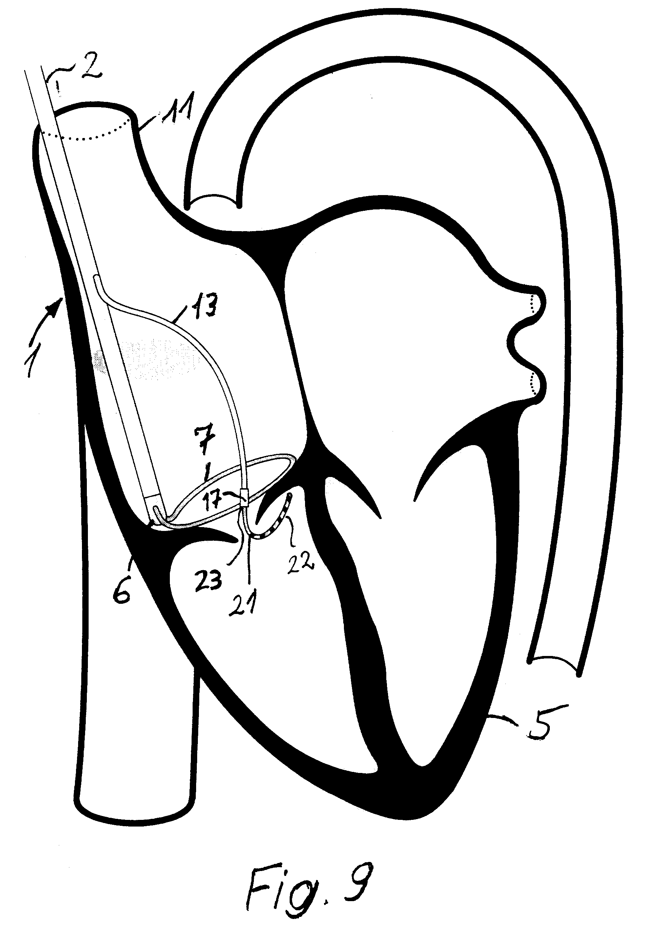

Es wurde bereits erwähnt, daß wenigstens ein weiterer Führungsdraht 13 vorgesehen sein kann und dies ist in den Ausführungsbeispielen gemäß Fig.6 bis 13 dargestellt und wird im folgenden näher erläutert. Dieser weitere Führungsdraht 13 ist gemäß den erwähnten Figuren in Kombination mit der Schlinge 7 vorgesehen, die als Halter für das distale Ende dieses weiteren Führungsdrahtes 13 dient, der ebenfalls aus dem Führungsschlauch 2 ausschiebbar ist, allerdings mit Abstand zu dem Ende 6 und zu der Schlinge 7, wie man es deutlich in den Figuren erkennt. Die Figuren 6, 7 und 8 machen deutlich, daß dadurch gewissermaßen ein aus weiterem Führungsdraht 13, Schlinge 7 und Katheter 1 bzw. Führungsschlauch 2 bestehendes Raumgebilde geschaffen wird, welches sich gut im Vorhof des Herzens 5 (Fig.6) oder in einer Herzkammer (Fig.7 und Fig.8) positionieren und festlegen läßt und eine punktuelle Messung an vielen Stellen der Herzinnenwand ermöglicht, bevor eine neue Positionierung erforderlich wird.

Dieser weitere Führungsdraht 13 tritt im Winkel zu der von der Schlinge 7 aufgespannten Ebene proximal zu dem Austritt der Schlinge 7 aus dem Führungsschlauch 2 - mit Abstand - aus und ist, wie schon erwähnt, ausschiebbar. In den Figuren 11 bis 13 erkennt man, daß dieser weitere Führungsdraht 13 mit einer Führung, die in diesem Falle als Öse 17 ausgebildet ist, an dem die Schlinge 7 bildenden Führungsdraht oder -faden angreift und darauf verschiebbar ist. Dabei kann die Öse 17 gegebenenfalls noch eine gewisse räumliche Ausdehnung haben und als kurze Hülse ausgebildet sein.

Zum Verschieben der Führung oder Öse 17 des weiteren Führungsdrahtes 13 und vor allem seines distalen Endes entlang der Schlinge 7 ist eine diese Führung oder Öse 17 beaufschlagende, über die Schlinge 7 schiebbare, biegbare Hülse oder Wendel vorgesehen, wobei diese schiebbare Hülse ein biegbarer Schlauch oder in besonders bevorzugter Form die mehrere Elektroden oder Pole aufweisende Mehrfachwendel 4 sein kann, wie es in den Figuren 10 bis 13 angedeutet ist. In diesem letzteren Falle stehen sowohl die Schlinge als auch der weitere Führungsdraht 13 dazu zur Verfügung, eine Mehrfachelektrode 4 relativ dazu zu verschieben und somit entsprechend viele Meßpunkte im Herzen bei einer Positionierung erreichen zu können.

Dabei hat dieser weitere Führungsdraht 13 einen Querschnitt, der gleich oder bevorzugt kleiner als der Innenquerschnitt eines inneren Kanales oder einer weiteren elektrischen Zuleitung 4 mit insbesondere mehreren Elektroden oder Polen, vorzugsweise die schon erwähnte Mehrfachwendel, ist, so daß die Verschiebung einer solchen als Mehrfachwendel ausgebildeten Mehrfachelektrode 4, die in Fig.6 gut erkennbar ist, problemlos möglich ist.

Damit mit Hilfe der Mehrfachelektrode 4 an deren Polen gegebenenfalls auch eine Ablation möglich ist, kann wenigstens eine dieser Elektroden oder Pole eine Zuleitung für Hochfrequenzenergie haben oder es kann gemäß Fig.4 ein zusätzlicher Hochfrequenzpol 18 zum Koagulieren von Gewebe vorgesehen sein, der in diesem Ausführungsbeispiel am distalen Ende der Mehrfachelektrode 4 angeordnet ist. Dabei erkennt man, daß ferner ein Temperaturfühler 19 am oder im Hochfrequenzpol 18 angeordnet ist, der zur Regelung der Hochfrequenz mit einem diesen Hochfrequenzpol 18 beaufschlagenden, in der Zeichnung nicht dargestellten Hochfrequenzgenerator über eine Leitung 20 verbunden ist. Diese bei einer Mehrfachelektrode 4 im Zusammenhang mit der Schlinge 7 dargestellte Anordnung kann auch bei einer Mehrfachelektrode 4 vorgesehen sein, die über einen weiteren Führungsdraht 13 geschoben wird.

Für die erwähnte gute Anpassung der Schlinge 7 und/oder weiterer Führungsdrähte 13 sind diese aus flexibel biegsamem, an die Herzinnenseite und die dort herrschende Anatomie anpaßbarem, nicht bleibend verformbarem Draht, zum Beispiel aus Nitinol, oder aus Kunststoff gefertigt. Nitinol ist jedoch zu bevorzugen, weil dies ein geschmeidiger flexibler Werkstoff ist, der sich gut unter entsprechender Druckbelastung an anatomische Formen anpaßt, aber beim Zurückziehen auch elastisch wieder in seiner ursprüngliche Form zurückverformt.

In Fig.8 ist ein Ausführungsbeispiel dargestellt, bei welchem zwei Schlingen 7 vorgesehen sind, wobei die Ebenen, in denen diese Schlingen angeordnet sind, in einem Winkel zueinander stehen. Die Positionierung und vor allem die Festlegung des distalen Endes des Katheters innerhalb einer Herzkammer wird dadurch, wie in Fig.8 gut erkennbar, weiter verbessert, wobei zu bedenken ist, daß das Herz 5 durch seine Pumpleistung in ständiger Bewegung ist. Die zusätzliche Verankerung mit einer weiteren Schlinge 7 ist vor allem dann vorteilhaft, wenn nahe der Herzspitze Messungen durchgeführt werden sollen.

Dabei erkennt man in Fig.8 ferner, daß die beiden Schlingen 7 etwa an derselben Stelle des Katheters 1 und nach derselben Seite hin unter verschiedenen Winkeln austreten. Zwar wäre auch ein Austritt nach verschiedenen Seiten denkbar, jedoch würde dies beim Fixieren des Katheters 1 weniger vorteilhaft sein, als wenn beide Schlingen 7 nach derselben Seite hin austreten und somit die Austrittsstelle der Schlingen des Katheters 1 entsprechend mehrfach nach derselben Seite hin abgestützt wird.

In diesem Ausführungsbeispiel ist die unter einem kleineren Winkel gegenüber dem Katheter 1 geneigte, dem Katheterende somit nähere Schlinge 7 als Halter für den weiteren Führungsdraht 13 oder ein auf der Schlinge 7 verschiebbares Halteelement für den weiteren Führungsdraht 13 ausgebildet. Zwar könnte eine Anordnung auch darin bestehen, daß nur zwei Schlingen vorgesehen sind, jedoch ist in diesem Falle auch ein weiterer Führungsdraht 13 vorgesehen.

Während die Figuren 10 und 11 darstellen, wie ein weiterer Führungsdraht 13 mit einer Schlinge 7 verbunden und gemäß dem Doppelpfeil Pf1 in Fig.11 darauf verschiebbar sein kann, zeigen die Figuren 12 und 13 einer Anordnung, bei welcher zwei weitere Führungsdrähte 13 vorgesehen sind. Deren dem Katheter 1 und dem Führungsschlauch 2 abgewandten Enden sind dabei an derselben Schlinge 7 gehalten und zwar gemäß Fig.13 und dem Doppelpfeil Pf2 verschiebbar. Die Angriffsstellen oder Führungsösen 17 haben dabei einen Abstand zueinander, so daß eine noch bessere räumliche Verteilung von Führungsdrähten erreicht wird, auf denen entsprechende Mehrfachwendeln mit mehreren Elektroden oder Polen verschoben werden können.

Denkbar wäre auch, daß bei dieser Anordnung gemäß Fig.12 und 13 analog der Fig.8 zwei Schlingen 7 vorgesehen sind. Die weiteren beiden Führungsdrähte 13 könnten dann an der dem Katheterende näheren Schlinge in der in Fig.13 dargestellten Weise angreifen, jedoch wäre es auch möglich, daß die beiden weiteren Führungsdrähte 13 jeweils an verschiedenen Schlingen 7 insbesondere verschiebbar angreifen.

Eine weitere Ausgestaltungsmöglichkeit zeigt das Ausführungsbeispiel gemäß Fig.9. In diesem Falle ist der weitere Führungsdraht 13 - der gemäß der vorstehenden Beschreibung auch mehrfach vorhanden sein könnte - als hohler Katheter ausgebildet und hat ein offenes Ende 23. Somit kann durch diesen hohlen Katheter eine Elektrodenzuleitung 21 mit einer oder mehreren Elektroden oder Polen an der Halterung oder Öse 17 dieses hohlen Katheters vorbei verschoben werden. Dabei macht Fig.9 deutlich, daß diese durch den hohlen Katheter ausschiebbare Elektrodenzuleitung 21 ein umgebogenes freies Ende 22 hat und etwa J-förmig gestaltet ist, so daß das freie, die Pole oder Elektroden aufweisende Ende 22 dieser Elektrodenzuleitung 21 an der sich auf den Herzklappen abstützenden Schlinge 7 vorbei verschiebbar ist und mit dem Ende 22 die Unterseite der Herzklappen erreichen kann, die bisher kaum oder nur sehr schwer erreichbar ist. Durch die gute Fixierung mit Hilfe der Schlinge 7 ist durch diese Anordnung und Ausführungsform diese schwer zugängliche Unterseite der Herzklappen ebenfalls erreichbar, so daß auch dort entsprechende Messungen und eventuell eine Ablation durchgeführt werden können.

Der Katheter 1 mit einem Führungsschlauch 2 und mit einer oder mehreren elektrischen Zuleitungen 3 zu einer Elektrode, insbesondere zu einer mehrere gegeneinander isolierte Pole aufweisenden Mehrfachelektrode 4 hat an dem in das Herz 5 einführbaren distalen Ende 6 wenigstens eine Schlinge 7 aus biegsamem oder flexiblem Werkstoff, die als Stützschlinge dient und durch Ausschieben aus dem Führungsschlauch 2 des Katheters 1 in ihrem Umfang vergrößert oder verkleinert werden kann. Sie kann als Führungsdraht für die Elektrode 4 dienen, so daß entlang ihrer Abstützung an der Innenseite des Herzens entsprechende Messungen oder eine Therapie durchgeführt werden können. Sie kann aber zusätzlich oder stattdessen auch als Halter für wenigstens einen weiteren, ebenfalls aus dem Führungsschlauch 2 ausschiebbaren weiteren Führungsdraht 13 für eine Elektrode oder Mehrfachelektrode 4 dienen. Trotz des ständig schlagenden Herzens kann also der Katheter 1 mit der Elektrode 4 gut und schnell positioniert und verankert werden, um entsprechende Herzsignale oder elektrische Potentiale im Herzen 5 messen zu können.

## Patentansprüche

1. Katheter (1) mit einem Führungsschlauch (2) und mit einer oder mehreren elektrischen Zuleitungen (3) zu einer Elektrode oder zu einer mehrere gegeneinander isolierte Pole aufweisenden Mehrfachelektrode (4), die zum intrakardialen, insbesondere punktuellen Messen oder Aufnehmen von Herzsignalen oder elektrischen Potentialen im Herzen (5) und/oder zum Stimulieren dient, **dadurch gekennzeichnet**, daß an dem in das Herz (5) einführbaren distalen Ende (6) des Katheters (1) wenigstens eine Schlinge (7) aus biegsamem oder flexiblem Werkstoff angeordnet und durch Ausschieben aus dem Führungsschlauch (2) des Katheters (1) in ihrem Umfang vergrößerbar ist und daß diese Schlinge (7) als Führungsdraht oder -faden und/oder als Halter für wenigstens einen ebenfalls aus dem Führungsschlauch (2) ausschiebbaren weiteren Führungsdraht (13) für die Elektrode oder Mehrfachelektrode (4) dient.

2. Katheter nach Anspruch 1, dadurch gekennzeichnet, daß die Schlinge und/oder der weitere Führungsdraht (13) stufenlos verschiebbar und dadurch in ihrer wirksamen Länge stetig vergrößerbar sind.

3. Katheter nach Anspruch 1 oder 2, dadurch gekennzeichnet, daß die Elektrode oder Mehrfachelektrode (4) eine Wendel aus einem oder mehreren Drähten ist, die in ihrem Inneren einen Kanal für den Führungsdraht freilassen, gegeneinander isoliert sind und am Endbereich abisolierbare oder abisolierte Stellen haben, wobei der Querschnitt des inneren Kanales der Elektrode oder Mehrfachelektrode (4) gleich oder größer als der Außenquerschnitt des die Schlinge (7) bildenden Drahtes oder Fadens oder des weiteren Führungsdrahtes (13) ist, und daß die Wendel (4) und insbesondere ihr Ende auf der Schlinge (7) und/oder dem weiteren Führungsdraht (13) verschiebbar ist.

4. Katheter nach einem der Ansprüche 1 bis 3, dadurch gekennzeichnet, daß die Schlinge (7) mit einem Ende (8) an der Innenseite des Führungsschlauches (2) des Katheters (1) befestigt ist und ihr anderes Ende (9) durch den Katheter und dessen Führungsschlauch (7) zum proximalen Katheterende verläuft und daß die Elektrode oder Mehrfachelektrode (4) von dem proximalen Katheterende aus bis zu der Befestigungsstelle (12) der Schlinge (7) verschiebbar ist.

5. Katheter nach einem der Ansprüche 1 bis 4, dadurch gekennzeichnet, daß die Schlinge (7) insbesondere an ihrer distalen Befestigungsstelle eine Vorspannung oder Verbiegung hat, durch welche die von der Schlinge (7) aufgespannte Ebene in einem Winkel zum Katheterverlauf, insbesondere in einem stumpfen Winkel, angeordnet ist.

6. Katheter nach einem der Ansprüche 1 bis 5, dadurch gekennzeichnet, daß am distalen Ende (6) des Führungsschlauches (2) ein Austrittskanal (15) für das verschiebbare Ende der Schlinge (7) exzentrisch und etwa auf einem Durchmesser gegenüberliegend eine Eintrittsöffnung (16) mit der Befestigungsstelle (12) für das feste Ende (8) der Schlinge (7) angeordnet sind.

7. Katheter nach einem der Ansprüche 1 bis 6, dadurch gekennzeichnet, daß der weitere Führungsdraht (13) im Winkel zu der von der Schlinge (7) aufgespannten Ebene proximal zu dem Austritt der Schlinge (7) aus dem Führungsschlauch (2) austritt und ausschiebbar ist und mit einer Führung, insbesondere einer Öse (17), an dem die Schlinge (7) bildenden Führungsdraht oder -faden angreift und/oder darauf verschiebbar ist.

8. Katheter nach Anspruch 7, dadurch gekennzeichnet, daß zum Verschieben der Führung oder Öse (17) des weiteren Führungsdrahtes (13) entlang der Schlinge (7) eine diese Führung beaufschlagende, über die Schlinge (7) schiebbare, biegbare Hülse oder Wendel vorgesehen ist.

9. Katheter nach Anspruch 8, dadurch gekennzeichnet, daß die über die Schlinge schiebbare Hülse ein biegbarer Schlauch ist.

10. Katheter nach einem der Ansprüche 8 oder 9, dadurch gekennzeichnet, daß die zum Verstellen des weiteren Führungsdrahtes dienende Hülse oder Wendel ihrerseits mehrere Elektroden oder Pole aufweist oder als Mehrfachwendel mit mehreren gegeneinander isolierten Elektroden (4) oder Polen ausgebildet ist.

11. Katheter nach einem der Ansprüche 1 bis 10, dadurch gekennzeichnet, daß der weitere Führungsdraht (13) einen Querschnitt hat, der gleich oder kleiner als der Innenquerschnitt eines inneren Kanales in einer weiteren elektrischen Zuleitung mit insbesondere mehreren Polen, vorzugsweise einer Mehrfachwendel, ist.

12. Katheter nach einem der Ansprüche 1 bis 11, dadurch gekennzeichnet, daß wenigstens einer der Pole eine Zuleitung für Hochfrequenzenergie hat oder ein zusätzlicher Hochfrequenzpol zum Koagulieren von Gewebe vorgesehen ist.

13. Katheter nach Anspruch 12, dadurch gekennzeichnet, daß wenigstens ein Temperaturfühler am oder im Hochfrequenzpol (18) angeordnet ist und insbesondere zur Regelung der Hochfrequenz mit einem diesen Hochfrequenzpol (18) beaufschlagenden Hochfrequenzgenerator verbunden ist.

14. Katheter nach einem der Ansprüche 1 bis 13, dadurch gekennzeichnet, daß die Schlinge und/oder der oder die daran gehaltenen Führungsdrähte aus flexibel biegsamem, an die Herzinnenseite anpaßbarem, nicht bleibend verformbarem Draht, zum Beispiel aus Nitinol oder aus Kunststoff besteht.

15. Katheter nach einem der Ansprüche 1 bis 14, dadurch gekennzeichnet, daß zwei Schlingen (7) vorgesehen sind und daß die Ebenen, in denen diese Schlingen (7) angeordnet sind, in einem Winkel zueinander stehen.

16. Katheter nach Anspruch 15, dadurch gekennzeichnet, daß die beiden Schlingen (7) etwa an derselben Stelle des Katheters (1) und nach derselben Seite hin unter verschiedenen Winkeln austreten.

17. Katheter nach Anspruch 15 oder 16, dadurch gekennzeichnet, daß die unter einem kleineren Winkel gegenüber dem Katheter (1) geneigte, dem Katheterende nähere Schlinge (7) als Halter für den weiteren Führungsdraht (13) oder ein auf der Schlinge (7) verschiebbares Halteelement für den weiteren Führungsdraht (13) dient.

18. Katheter nach einem der Ansprüche 1 bis 17, dadurch gekennzeichnet, daß wenigstens zwei weitere Führungsdrähte (13) vorgesehen sind, deren dem Katheter abgewandte Enden an derselben Schlinge oder verschiedenen Schlingen gehalten, insbesondere verschiebbar gehalten sind und deren Angriffsstellen an der/den Schlingen einen Abstand zueinander haben.

19. Katheter nach einem der Ansprüche 1 bis 18, dadurch gekennzeichnet, daß der weitere Führungsdraht (13) oder wenigstens einer von mehreren weiteren Führungsdrähten als hohler Katheter ausgebildet ist und ein offenes Ende (23) hat, so daß durch diesen hohlen Katheter eine Elektrodenzuleitung mit einer oder mehreren Elektroden oder Polen an der Halterung dieses hohlen Katheters vorbei verschiebbar ist.

20. Katheter nach Anspruch 19, dadurch gekennzeichnet, daß die durch den hohlen Katheter ausschiebbare Elektrodenzuleitung (21) ein umgebogenes freies Ende (22) hat und etwa J-förmig gestaltet ist, so daß das freie, die Pole oder Elektroden aufweisende Ende (22) dieser Elektrodenzuleitung (21) an der sich auf den Herzklappen abstützenden Schlinge (7) vorbei verschiebbar ist und mit dem Ende (22) die Unterseite der Herzklappen erreichbar ist.

## Claims

1. Catheter (1) having a guide tube (2) and one or more electric leads (3) to an electrode or to a multiple electrode (4) having a plurality of poles insulated from one another, said electrode (4) being used for intracardiac, particularly spot measurement or recording of heart signals or electrical potential in the heart (5) and/or for stimulation, characterised in that, at a distal end (6) of the catheter (1) which can be inserted into the heart (5), there is at least one loop (7) of bendable or flexible material which can be enlarged in circumference by pushing it out of the guide tube (2) of the catheter (1), and in that this loop (7) acts as a guide wire or filament and/or as a holder for at least one other guide wire (13) for the electrode or multiple electrode (4) which can also be pushed out of the guide tube (2).

2. Catheter according to claim 1, characterised in that the loop and/or the additional guide wire (13) can be pushed smoothly along and its effective length can thereby be steadily enlarged.

3. Catheter according to claim 1 or 2, characterised in that the electrode or multiple electrode (4) is a coil of one or more wires which leave a channel free for the guide wire inside them, are insulated from one another and in their end portion have insulated or insulatable points, the cross section of the inner channel of the electrode or multiple electrode (4) being equal to or greater than the external cross section of the wire or filament or additional guide wire (13) which forms the loop (7), and in that the coil (4) and particularly its end is movable on the loop (7) and/or the additional guide wire (13).

4. Catheter according to one of claims 1 to 3, characterised in that the loop (7) is secured at one end (8) to the inside of the guide tube (2) of the catheter (1) and its other end (9) extends through the catheter and its guide tube (7) to the proximal end of the catheter and in that the electrode or multiple electrode (4) is movable from the proximal end of the catheter up to the fixing point (12) of the loop (7).

5. Catheter according to one of claims 1 to 4, characterised in that the loop (7) has, particularly at its distal fixing point, a bias or bend by means of which the plane spanned by the loop (7) is arranged at an angle, particularly an obtuse angle, to the direction of the catheter.

6. Catheter according to one of claims 1 to 5, characterised in that at the distal end (6) of the guide tube (2) an exit channel (15) for the movable end of the loop (7) is eccentrically arranged and more or less diametrically opposite is an entry opening (16) with the fixing point (12) for the fixed end (8) of the loop (7).

7. Catheter according to one of claims 1 to 6, characterised in that the additional guide wire (13) emerges from and can be pulled out from the guide tube (2) at an angle to the plane spanned by the loop (7), proximally with respect to the exit of the loop (7), and engages on and/or is movable on the guide wire or filament forming the loop (7), by means of a guide, particularly an eyelet (17).

8. Catheter according to claim 7, characterised in that, in order to move the guide or eyelet (17) of the additional guide wire (13) along the loop (7), a bendable sleeve or coil is provided which acts on this guide and is slidable over the loop (7).

9. Catheter according to claim 8, characterised in that the sleeve which can be slid over the loop is a bendable tube.

10. Catheter according to one of claims 8 or 9, characterised in that the sleeve or coil used to adjust the additional guide wire in turn has a plurality of electrodes or poles or is constructed as a multiple coil with a plurality of electrodes (4) or poles insulated from one another.

11. Catheter according to one of claims 1 to 10, characterised in that the additional guide wire (13) has a cross section which is equal to or less than the internal cross section of an inner channel in another electric lead having, in particular, a plurality of poles, preferably a multiple coil.

12. Catheter according to one of claims 1 to 11, characterised in that at least one of the poles has a lead for supplying high frequency energy or an additional high frequency pole is provided for coagulating tissue.

13. Catheter according to claim 12, characterised in that at least one temperature sensor is provided on or in the high frequency pole (18) and, particularly for regulating the high frequency, is connected to a high frequency generator which acts upon this high frequency pole (18).

14. Catheter according to one of claims 1 to 13, characterised in that the loop and/or the guide wire or wires held thereon comprise(s) flexibly bendable wire which is not permanently deformable and which can be adapted to the inside of the heart, this wire consisting, for example, of nitinol or plastics.

15. Catheter according to one of claims 1 to 14, characterised in that two loops (7) are provided and the planes in which these loops (7) are arranged are at an angle to one another.

16. Catheter according to claim 15, characterised in that the two loops (7) emerge more or less at the same point of the catheter (1) and on the same side at different angles.

17. Catheter according to claim 15 or 16, characterised in that the loop (7) closer to the end of the catheter and inclined at a smaller angle to the catheter (1) acts as a holder for the additional guide wire (13) or as a retaining element, movable along the loop (7), for the additional guide wire (13).

18. Catheter according to one of claims 1 to 17, characterised in that at least two additional guide wires (13) are provided, the ends of which, remote from the catheter, are held, especially movably held, on the same loop or on different loops, and the points of attack of said wires on the loop or loops are spaced from one another.

19. Catheter according to one of claims 1 to 18, characterised in that the additional guide wire (13) or at least one of several additional guide wires is constructed as a hollow catheter and has an open end (23), so that, through this hollow catheter, an electrode lead with one or more electrodes or poles can be pushed past the mounting of this hollow catheter.

20. Catheter according to claim 19, characterised in that the electrode lead (21) which can be pushed out through the hollow catheter has a bent free end (22) and is substantially J-shaped, so that the free end (22) of this electrode lead (21) which carries the poles or electrodes can be pushed past the loop (7) resting on the heart valve and the underside of the heart valve can be reached with the end (22).

## Revendications

1. Cathéter (1) avec un tuyau de guidage (2) et avec une ou plusieurs lignes d'alimentation électriques (3) en direction d'une électrode ou d'une électrode multiple (4) présentant plusieurs électrodes ou pôles isolés les uns des autres, et servant à effectuer notamment des mesures ponctuelles intracardiaques ou à enregistrer des signaux cardiaques ou des potentiels électriques dans le coeur (5) et/ou à le stimuler, **caractérisé en ce qu**'au moins une boucle (7) en matière souple ou flexible est placée à l'extrémité distale (6) du cathéter (1) pouvant être introduite dans le coeur (5), la circonférence de cette boucle (7) pouvant être agrandie en poussant la boucle hors du tuyau de guidage (2) du cathéter (1), et en ce que cette boucle (7) sert de fil métallique de guidage (13) ou de fil de guidage et/ou de support pour au moins un fil de guidage supplémentaire (13) pouvant être également poussé hors du tuyau de guidage (2) pour l'électrode ou l'électrode multiple (4).

2. Cathéter selon la revendication 1, **caractérisé en ce que** la boucle et/ou l'autre fil métallique de guidage (13) sont déplaçables progressivement et que leur longueur effective peut donc être agrandie de façon continue.

3. Cathéter selon la revendication 1 ou 2, **caractérisé en ce que** l'électrode ou l'électrode multiple (4) peut de façon utile être une hélice à un ou plusieurs fils qui laissent libre un canal intérieur pour le fil de guidage, isolés les uns des autres, et ont des endroits pouvant être dénudés ou des endroits dénudés dans la partie terminale, la section du canal intérieur de l'électrode ou de l'électrode multiple (4) étant égale ou supérieure à la section extérieure du fil métallique formant la boucle (7) ou du fil ou du fil métallique de guidage supplémentaire (13), et en ce que l'hélice (4), en particulier son extrémité, peut être déplacée sur la boucle (7) et/ou le fil métallique de guidage supplémentaire (13).

4. Cathéter selon l'une des revendications 1 à 3, **caractérisé en ce que** la boucle (7) est fixée par l'une des extrémités (8) à la paroi interne du tuyau de guidage (2) du cathéter (1) et que son autre extrémité (9) passe par le cathéter et le tuyau de guidage (2) de celui-ci vers l'extrémité proximale du cathéter, et en ce que l'électrode ou l'électrode multiple (4) est déplaçable depuis l'extrémité proximale du cathéter jusqu'à l'endroit de fixation (12) de la boucle (7).

5. Cathéter selon l'une des revendications 1 à 4, **caractérisé en ce que** la boucle (7) a une prétension ou une courbure, notamment à son endroit de fixation distale, grâce à laquelle le niveau formé par la boucle (7) est placé en angle par rapport à l'allure du cathéter, notamment en angle obtus.

6. Cathéter selon l'une des revendications 1 à 5, **caractérisé en ce qu**'un canal de sortie (15) est placé de façon excentrique à l'extrémité distale (6) du tuyau de guidage (2) pour l'extrémité déplaçable de la boucle (7) et qu'une ouverture d'entrée (16) est placée à peu près sur un diamètre de manière opposée avec l'endroit de fixation (12) de l'extrémité fixe (8) de la boucle (7).

7. Cathéter selon l'une des revendications 1 à 6, **caractérisé en ce que** le fil métallique de guidage supplémentaire (13) sort du tuyau de guidage (2) en angle par rapport au niveau formé par la boucle (7) et de manière proximale par rapport à la sortie de la bouche (7) et qu'il peut être déplacé vers l'extérieur, et qu'il agit au moyen d'un guidage, notamment un oeillet (17), sur le fil métallique et/ou fil de guidage formant la bouche et/ou déplaçable sur celui-ci.

8. Cathéter selon la revendication 7, **caractérisé en ce que** pour déplacer le guidage ou l'oeillet (17) de l'autre fil métallique de guidage (13) le long de la boucle (7), on peut prévoir une douille ou une hélice flexible agissant sur ce guidage et pouvant être déplacée sur la boucle (7).

9. Cathéter selon la revendication 8, **caractérisé en ce que** la douille déplaçable sur la boucle est un tuyau flexible.

10. Cathéter selon l'une des revendications 8 ou 9, **caractérisé en ce que** la douille ou l'hélice servant à régler l'autre fil métallique de guidage présente de son côté plusieurs électrodes ou pôles, ou est formée comme hélice multiple avec plusieurs électrodes (4) ou pôles isolés les uns des autres.

11. Cathéter selon l'une des revendications 1 à 10, **caractérisé en ce que** l'autre fil métallique de guidage (13) a une section égale ou inférieure à la section intérieure d'un canal intérieur dans une autre ligne d'alimentation électrique avec notamment plusieurs pôles, et est de préférence une hélice multiple.

12. Cathéter selon l'une des revendications 1 à 11, **caractérisé en ce qu**'au moins un des pôles a une ligne d'alimentation pour l'énergie à haute fréquence ou qu'un autre pôle à haute fréquence est prévu pour la coagulation de tissu.

13. Cathéter selon la revendication 12, **caractérisé en ce qu**'au moins un capteur de température est placé sur ou dans le pôle à haute fréquence (18), et notamment en ce que, pour le réglage de la haute fréquence, il est raccorde à un générateur à haute fréquence agissant sur l'un de ces pôles à haute fréquence (18).

14. Cathéter selon l'une des revendications 1 à 13, **caractérisé en ce que** la boucle et/ou le ou les fils métalliques de guidage qui y sont maintenus sont fabriqués en fil flexible souple pouvant s'ajuster à l'intérieur du coeur, et non déformables en permanence, par exemple en Nitinol, ou en plastique.

15. Cathéter selon l'une des revendications 1 à 14, **caractérisé en ce que** deux boucles (7) sont prévues et que les niveaux dans lesquels sont placées ces boucles (7) sont angulaires l'un par rapport à l'autre.

16. Cathéter selon la revendication 15, **caractérisé en ce que** les deux boucles (7) sortent à peu près au même endroit du cathéter (1) et du même côté sous différents angles.

17. Cathéter selon la revendication 15 ou 16, **caractérisé en ce que** la boucle (7) la plus proche de l'extrémité du cathéter, dont l'angle d'inclinaison est plus petit par rapport au cathéter (1), sert d'appui pour l'autre fil métallique de guidage (13) ou d'élément d'appui déplaçable sur la boucle pour l'autre fil métallique de guidage (13).

18. Cathéter selon l'une des revendications 1 à 17, **caractérisé en ce qu**'au moins deux fils métalliques de guidage supplémentaires (13) sont prévus, dont les extrémités à l'opposé du cathéter sont maintenues sur la même boucle ou sur différentes boucles, notamment de façon déplaçable, et dont les endroits de prise à la/aux boucle(s) ont un écart mutuel.

19. Cathéter selon l'une des revendications 1 à 18**, caractérisé en ce que** le fil métallique de guidage supplémentaire (13) ou au moins l'un de plusieurs fils métalliques de guidage supplémentaires est formé comme cathéter creux et a une extrémité ouverte (23), de sorte qu'une ligne d'alimentation d'électrode avec une ou plusieurs électrodes ou pôles est déplaçable dans ce cathéter creux devant le support de ce cathéter creux.

20. Cathéter selon la revendication 19, **caractérisé en ce que** la ligne d'alimentation d'électrode (21) déplaçable dans le cathéter creux a une extrémité libre recourbée (22) et est à peu près en forme de J, de sorte que l'extrémité libre (22) présentant les pôles ou les électrodes de cette ligne d'alimentation d'électrode (21) est déplaçable devant la boucle (7) s'appuyant sur la valvule et que le dessous de la valvule peut être atteint avec l'extrémité (22).
